# EUROPÄISCHE PATENTSCHRIFT

(11) **EP 1 276 537 B1**
(45) Veröffentlichungstag und Bekanntmachung des Hinweises auf die Patenterteilung: **05.09.2007**
(21) Anmeldenummer: 00991189.2
(22) Anmeldetag: 14.12.2000
(51) Int. Cl.: A61N 1/05

(54) **MIKROKONTAKTSTRUKTUR FÜR NEUROPROTHESEN ZUR IMPLANTATION AN NERVENGEWEBE**
MICROCONTACT STRUCTURE FOR NEUROPROSTHESES FOR IMPLANTING ON NERVE TISSUE
STRUCTURE DE MICRO CONTACT POUR NEUROPROTHESES A IMPLANTER SUR DES TISSUS NERVEUX

(30) Priorität: 28.04.2000 DE 10020846
(43) Veröffentlichungstag der Anmeldung: 22.01.2003
(73) Patentinhaber: IMI Intelligent Medical Implants AG, 6304 Zug (CH)
(72) Erfinder: ECKMILLER, Rolf, 41460 Neuss (DE); HÜNERMANN, Ralph, 50931 Köln (DE); BECKER, Michael, 53115 Bonn (DE)
(74) Vertreter: Graf von Stosch, Andreas
(86) Internationale Anmeldenummer: PCT/EP2000/012713
(87) Internationale Veröffentlichungsnummer: WO 2001/083025

(56) Entgegenhaltungen:
- WO-A-97/24156
- DE-A- 4 424 753
- US-A- 4 940 065
- US-A- 5 215 088
- US-A- 5 324 322
- US-A- 5 919 220

## Beschreibung

Die Erfindung betrifft eine implantierbare Mikrokontaktstruktur für Retina-Implantate zur Behandlung von Funktionsstörungen des Nervensystems zum Zwecke der reversiblen Verankerung an Nervengewebe.

### Stand der Technik

Es sind mehrere Mikrokontaktstrukturen für teilweise implantierte Neuroprothesen bekannt, deren räumliche Mikrokontaktanordnung durch eine starre vorgeformte Fläche festgelegt ist, so z.B. in der Patentschrift US 5,215,088.

Es sind mehrere Mikrokontaktstrukturen für teilweise implantierte Neuroprothesen bekannt, deren räumliche Mikrokontaktanordnung durch eine teilweise elastische, biegsame vorgeformte Fläche festgelegt ist und sich durch die Art der Implantatbefestigung sowie durch passive Anpassung an die Gewebeform im Implantatbereich verändern kann, so in der DE 4424753 A1.

Die Herstellung einer solchen Mikrokontaktstruktur ist beispielsweise aus der folgenden Veröffentlichung bekannt:
"Flexible, polyimide-based neural interfaces." Stieglitz T. Beutel H. Keller R. Schuettler M. Meyer J-U. Proceedings of the Seventh International Conference on Microelectronics for Neural, Fuzzy and Bio-Inspired Systems. IEEE Comput. Soc. 1999, pp.112-19. Los Alamitos, CA, USA.

Eine Mikrokontakstruktur gemäß Oberbegriff des Anspruchs 1 ist aus US-A- 5 919 220 bekannt.

### Nachteile des Standes der Technik

An den bekannten Mikrokontaktstrukturen ist nachteilig, daß keine Vorrichtungen und Verfahren für eine Explantation der Mikrokontaktstruktur vorgesehen sind.

Die bekannten Mikrokontaktstrukturen verfügen weiter nicht über Mechanismen, die die Anpassung der Mikrokontaktstruktur an die Form des zu kontaktierenden Gewebes vollziehen. Deswegen kann nicht sichergestellt werden, daß der Abstand zwischen den Mikroelektroden und den zu stimulierenden Neuronen des Nervengewebes minimal ist.

Nachteilig an den bekannten Mikrokontaktstrukturen ist weiter, daß sie nicht über die Möglichkeit der selbsttätigen Befestigung der Mikrokontaktstruktur am Nervengewebe verfügen.

Nachteilig an den gegenwärtig konzipierten bzw. verfügbaren Mikrokontaktstrukturen für epi-retinale Sehprothesen ist, daß diesen Merkmale fehlen, die die Einbringung in das Auge in räumlich komprimierter Form erlauben, und deshalb komplizierte chirurgische Techniken erforderlich sind. Diese Schwierigkeit wird sich in Zukunft verstärken, weil die räumlichen Abmessungen der Mikrokontaktstrukturen mit steigender Kontaktanzahl größer werden.

Ferner fehlt den gegenwärtig konzipierten bzw. verfügbaren Mikrokontaktstrukturen für epi-retinale Sehprothesen die Möglichkeit, die Neurone der Retina, die den Bereich des schärfsten Sehens vermitteln, mit einer hohen Mikrokontaktdichte zu belegen, da sich solche Neurone im sog. parafovealen Zellkrater befinden, der sich durch eine räumliche Kraterstruktur auszeichnet.

Es ist deshalb die Aufgabe der vorliegenden Erfindung, diese Nachteile zu beseitigen und eine Mikrokontaktstruktur zu offenbaren, die in komprimierter Form in den Körper einbringbar ist und reversibel am Nervengewebe verankert werden kann.

Diese Aufgabe wird von einer Mikrokontaktstruktur mit dem Merkmalen des Anspruchs 1 gelöst.

Die Mikrokontaktsstruktur gewährleistet einen guten Kontakt bzw. Wirkungszusammenhang mit Nervengewebe, da die implantierte Mikrokontaktstruktur relativ zueinander bewegliche Teilflächen umfaßt, die in mindestens zwei dauerhafte Vorzugsstellungen relativ zueinander gebracht werden können. und die während der Implantation in eine Vorzugsstellung zum Zwecke der mechanischen Verankerung mit dem zu kontaktierenden Nervengewebe gebracht sowie während der Explantation zur Lösung der Verankerung aus einer Vorzugsstellung in eine Andere gebracht werden können.

### Beschreibung von Ausführungsbeispielen

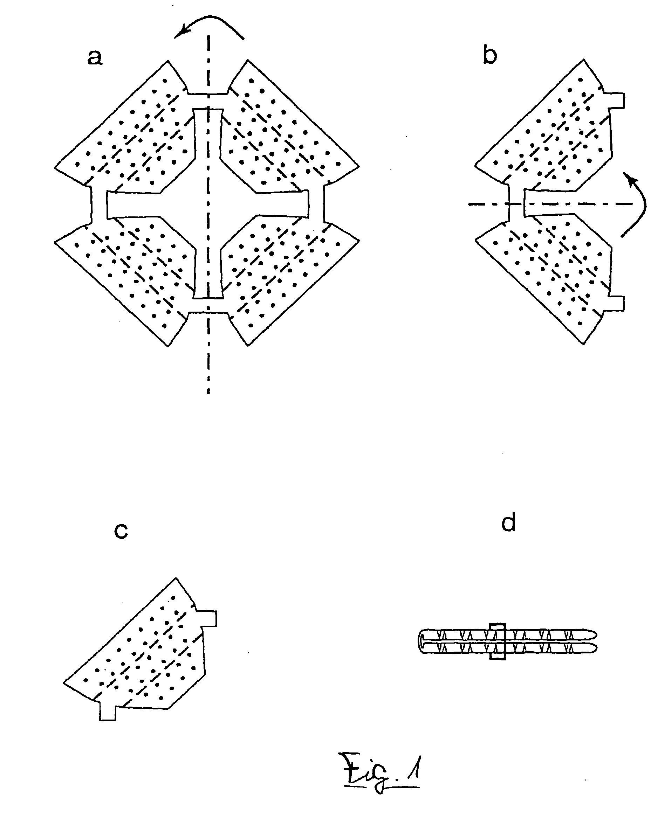

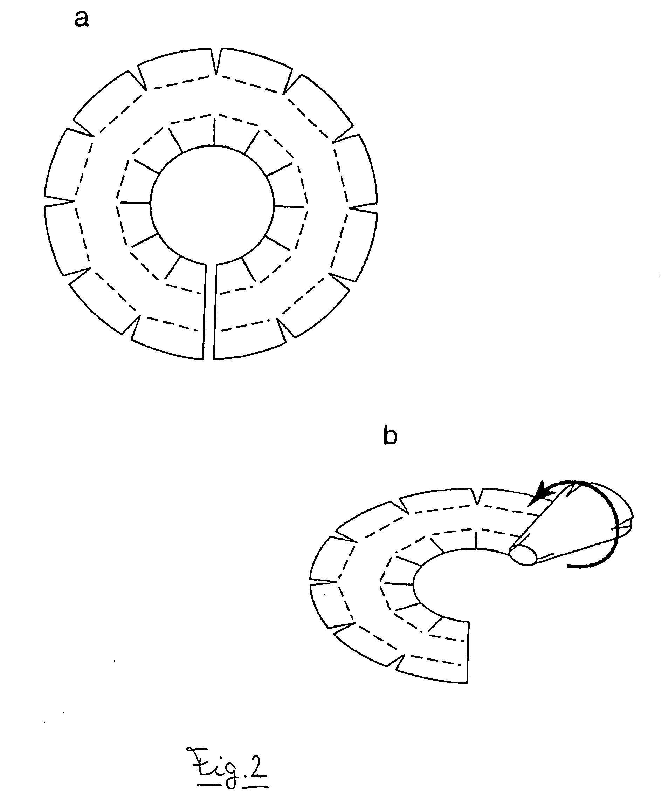

Vorteilhafte Ausgestaltungen der räumlich adaptiven Mikrokontaktstruktur sind anhand der Fig. 1 bis Fig. 4 dargestellt.

Eine vorteilhafte Ausgestaltung der Vorrichtung einer räumlich adaptiven Mikrokontaktstruktur für Neuroprothesen zur Implantation an Nervengewebe besteht darin, daß die Mikrokontaktstruktur als eine planare zweidimensionale Struktur in gängigen Verfahren zur Herstellung von Mikrokontaktstrukturen z.B. auf Silizium-, Silikon- oder Polyimid-Basis hergestellt werden kann (s. Fig. 1-4), in einem zweiten Schritt für Transportzwecke sehr kompakt gefaltet oder gerollt werden kann und in einem dritten Schritt nicht nur wieder planar entfaltet, sondern in die dritte Dimension gefaltet oder gerollt wird (s. Fig 1-3), so daß eine dreidimensionale Struktur entsteht.

Eine vorteilhafte Ausgestaltung der Mikrokontaktstruktur besteht darin, daß sie mit weiteren Modulen der Neuroprothese über Signalwege in Verbindung steht.

Eine vorteilhafte Ausgestaltung der Mikrokontaktstruktur besteht darin, daß auf der nach Implantation dem Nervengewebe zugewandten Seite hervorstehende Strukturen z.B. in Form von Mikroelektroden, Sensoren, Kanülen, oder Nägeln vorgesehen sind, die für die mechanische Verankerung der Mikrokontaktstruktur wesentlich sind.

Eine vorteilhafte Ausgestaltung der Mikrokontaktstruktur besteht darin, daß zum Zwecke der Überführung in eine Transportstellung durch Faltung, Rollung, oder Schachtelung der miteinander verbundenen Teile, Segmente bzw. Inseln aus einer vorgegebenen planaren Grundstellung Federelemente wie z.B. nach Art von Biegefedern oder Spiralfedern sowie elastische Elemente wie z.B. mit Gasen oder Flüssigkeiten gefüllte und mit einem elastischen Material umhüllte kissenähnliche Mikrostrukturen sowie z.B. poröse, schwammähnliche Mikrostrukturen gespannt werden so daß die automatische Wiederherstellung der Grundstellung hauptsächlich durch eine Transportsicherung verhindert wird.

Eine vorteilhafte Ausgestaltung der Transportsicherung besteht darin, daß die Mikrokontaktstruktur in der Transportstellung durch eine die Kräfte aufnehmende Klammer, oder Hülle, oder Verstiftung wird.

Eine vorteilhafte Ausgestaltung der Bedienung der Transportsicherung besteht darin, daß die Transportsicherung am Implantationsort durch Verwendung eines geeigneten Werkzeuges so gelöst wird, daß die Überführung aus der Transport- in die Grundstellung als kontrollierte Bewegung erfolgt. Dies erfolgt im Falle von Klammer, Hülle oder Stift vorzugsweise dadurch, daß die Kräfte zunächst von einem z.B. zangenähnlichen Werkzeug aufgenommen werden, daß anschließend die Transportsicherung mit einem weiteren Werkzeug entfernt und daß anschließend die Überführung in die Grundstellung mit Hilfe des zangenähnlichen Werkzeuges kontrolliert wird.

Die Überführung von gesicherter Transportstellung in Grundstellung erfolgt in einer weiteren vorteilhaften Ausgestaltung im Falle einer durch Temperaturabsenkung realisierten Hülle bzw. durch Vereisung erzeugten Bewegungsblockade vorzugsweise dadurch, daß durch eine geeignet geformte und steuerbare lokale Wärmequelle entweder als separates Werkzeug oder als integriertes Element der der Mikrokontaktstruktur nach Positionierung der Mikrokontaktstruktur am Implantationsort die Beweglichkeit seiner Teile durch kontrollierte Wärmezufuhr wieder hergestellt wird.

Eine vorteilhafte Ausgestaltung der Mikrokontaktstruktur besteht darin, daß die Rück-Überführung von Betriebsstellung in Grundstellung im Falle einer durch Temperaturabsenkung realisierten Hülle bzw. durch Vereisung erzeugten Bewegungsblockade vorzugsweise dadurch, daß durch eine geeignet geformte und steuerbare lokale Kältequelle (z.B. Peltier-Element) als integriertes Element der der Mikrokontaktstruktur zum Zwecke der Re-Explantation die Beweglichkeit seiner Teile durch kontrollierten Wärmeentzug blockiert wird.

Eine weitere vorteilhafte Ausgestaltung der Vorrichtung einer räumlich adaptiven Mikrokontaktstruktur besteht darin, daß die Struktur, die in einem zusammengefalteten Zustand gesichert transportiert und implantiert wird, sich bei Entfernung der Sicherung durch Materialeigenschaften selbst entfaltet und dadurch eine vorher eingeprägte dreidimensionale Struktur annimmt.

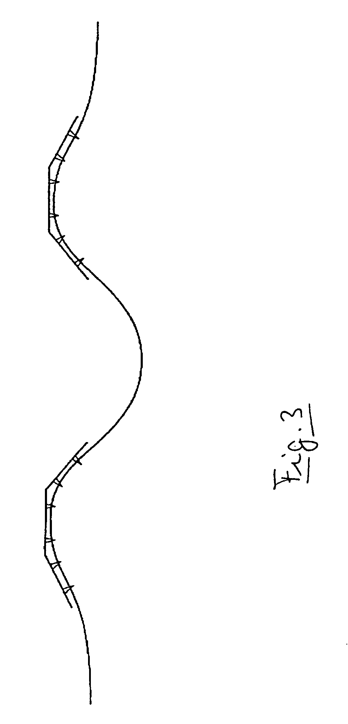

Eine weitere vorteilhafte Ausgestaltung der Vorrichtung einer räumlich adaptiven Mikrokontaktstruktur besteht darin, daß die Struktur durch die Selbstentfaltung eine Form annimmt, in der sie sich durch erhabene Mikrokontakte mit dem Gewebe verzahnen kann (s. Fig 3).

Eine weitere vorteilhafte Ausgestaltung der Vorrichtung einer räumlich adaptiven Mikrokontaktstruktur besteht darin, daß zum Zwecke der Explantation die verkürzten Verbindungen an der Struktur durchtrennt werden können und sich die Struktur durch Materialeigenschaften selbst wieder in einen planaren Zustand bringt, in dem sich die Verzahnungen mit dem Gewebe lösen.

Eine weitere vorteilhafte Ausgestaltung der Vorrichtung einer räumlich adaptiven Mikrokontaktstruktur besteht darin, daß die Struktur durch die Verzahnung mit dem Gewebe keine weitere Befestigung für eine ortsstabile Implantation benötigt.

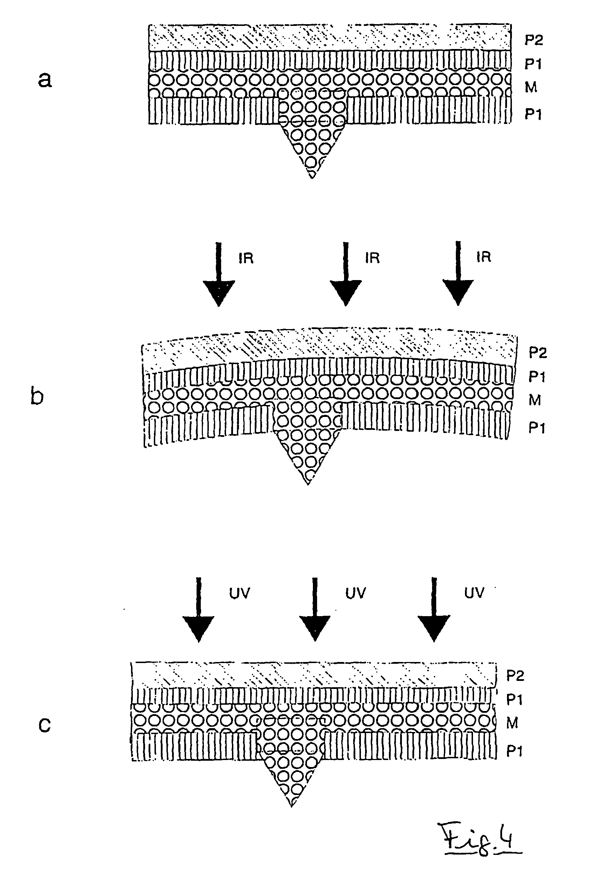

Eine weitere vorteilhafte Ausgestaltung des Verfahrens besteht darin, daß die Mikrokontaktstruktur auf einem mehrschichtig aufgebauten Substrat basiert, das über sogenannte Memory-Eigenschaften hinsichtlich der räumlichen Anordnung der Mikrokontaktstruktur verfügt. Die Fig. 4 zeigt einen Schnitt durch eine vorteilhafte 4-schichtige Mikrokontaktstruktur, bei der der Wirkungszusammenhang zwischen der Mikrokontaktstruktur und dem Nervengewebe durch elektrische Stimulation realisiert wird. Die dem zu stimulierenden Nervengewebe zugewandte Schicht besteht aus dem Polymer P1 (Polyimid) und enthält penetrierende Elektroden aus dem Metall M (Platin), das die anschließende Schicht bildet. Es folgt eine weitere Schicht des Polymers P1 und eine Schicht des Polymers P2 (Polyurethan). Das Polymer P2 hat die Eigenschaft der thermischen Ausdehnung relativ zu P1 und der Absorption von infraroter Strahlung (IR), so daß durch Bestrahlung mit IR-Licht eine definierte Volumenexpansion bewirkt wird.

Auf diese Weise wird die Mikrokontaktfolie durch fokussierte Bestrahlung an definierten Stellen verformt und an das Nervengewebe angepaßt. Weiterhin besitzt das Polymer P2 die Eigenschaft, bei der elektromagnetischen Bestrahlung aus dem ultravioletten Wellenlängenbereich strukturelle Übergänge zu vollziehen, die zur Kontraktion des Materials führen. Dadurch wird mittels fokussierter UV-Behandlung die Kompensation der zuvor durch IR-Licht erzielten Verformung bzw. die entgegengesetzte Verformung bewirkt, so daß die Ablösung der Mikrokontaktstruktur vom Nervengewebe erfolgt. Auf diese Weise wird die Re-Explantation der Mikrokontaktfolie eingeleitet.

## Patentansprüche

1. Implantierbare Mikrokontaktstruktur für Neuroprothesen zur Kontaktierung von Nervengewebe, mit einer Anzahl von Kontaktelementen, die auf wenigstens einem flächenhaften Träger ausgebildet sind, der wenigstens zwei relativ zueinander bewegliche Bereiche aufweist, die wenigstens zwei Vorzugsstellungen, nämlich eine Grundstellung und eine Betriebsstellung einnehmen können, wobei die Mikrokontaktstruktur für den Transport von oder zu einem Implantationsort in eine kompakte Grundstellung einstellbar ist, wobei die räumliche Ausdehnung der Mikrokontaktstruktur durch Faltung, Schachtelung oder Rollung der relativ zueinander beweglichen Bereiche verringert wird, und die Mikrokontaktstruktur zur mechanischen Verankerung sowie zur Kontaktierung des Nervengewebes am Implantationsort in eine Betriebsstellung mit einer planaren oder dreidimensionalen Struktur einstellbar ist, wobei die räumliche Ausdehnung der Mikrokontaktstruktur durch Öffnen der Faltung, Schachtelung oder Rollung der relativ zueinander beweglichen Bereiche vergrößert wird, **dadurch gekennzeichnet, daß** die Mikrokontaktstruktur eine Mikrokontaktstruktur zur retinalen Implantation für ein Retina-Implantat ist.

2. Mikrokontaktstruktur nach Anspruch 1 **dadurch gekennzeichnet, dass** die Mikrokontaktstruktur ein mehrschichtig aufgebautes Substrat umfasst, das Memory-Materialeigenschaften hinsichtlich der räumlichen Anordnung der Mikrokontaktstruktur aufweist.

3. Mikrokontaktstruktur nach Anspruch 2 **dadurch gekennzeichnet, dass** ein Wechsel oder eine Veränderung der Vorzugsstellungen der Mikrokontaktstruktur aufgrund der Memory-Materialeigenschaften der Mikrokontaktstruktur und durch externe Einwirkung vor der Implantation, durch eine externen Eingriff oder durch externe Signale aktiv steuerbar ist.

4. Mikrokontaktstruktur nach einem der vorhergehenden Ansprüche **dadurch gekennzeichnet, dass** die kompakte Grundstellung der Mikrokontaktstruktur für den chirurgischen Transport durch eine Transportsicherung gesichert wird, die durch einen externen Eingriff lösbar ist.

5. Mikrokontaktstruktur nach Anspruch 4 **dadurch gekennzeichnet, dass** die Transportsicherung der Mikrokontaktstruktur durch eine Klammer, Hülle oder Verstiftung ausgebildet ist.

6. Mikrokontaktstruktur nach einem der Ansprüche 4 oder 5 **dadurch gekennzeichnet, dass** sich die Mikrokontaktstruktur nach Entfernung der Transportsicherung aufgrund seiner Memory-Materialeigenschaften selbsttätig entfaltet und eine vorher im Material der Mikrokontaktstruktur eingeprägte dreidimensionale Struktur annimmt.

7. Mikrokontaktstruktur nach einem vorhergehenden Ansprüche **dadurch gekennzeichnet, dass** die Mikrokontaktstruktur erhabene Mikrokontakte aufweist, die sich in der Betriebsstellung mit dem zu kontaktierendem Gewebe verzahnen.

8. Mikrokontaktstruktur nach einem der vorhergehenden Ansprüche, **dadurch gekennzeichnet, dass** das Öffnen der Faltung, Schachtelung oder Rollung der relativ zueinander beweglichen Bereiche der Mikrokontaktstruktur nach dem Lösen der Transportsicherung aus der kompakten Grundstellung in eine zur Vorbereitung der mechanischen Verankerung geeignete Vorzugsstellung durch Freisetzung von Federkräften, molekularen Konformationsänderungen, pneumatischen Kräften, hydraulischen Kräften oder/und elektromagnetischen Kräften an den Übergängen zwischen den beweglichen Bereiche der Mikrokontaktstruktur selbsttätig erfolgt.

9. Mikrokontaktstruktur nach einem der vorhergehenden Ansprüche **dadurch gekennzeichnet, dass** der Wechsel oder die Veränderung einer Vorzugsstellung der Mikrokontaktstruktur zum Zwecke seiner mechanischen Verankerung am Nervengewebe durch eine extern festgelegte Einwirkung in einem zeitlich kontrollierten Ablauf bezüglich Bewegung und Kraft einstellbar ist.

10. Mikrokontaktstruktur nach einem der vorhergehenden Ansprüche **dadurch gekennzeichnet, dass** der Wechsel oder die Veränderung einer Vorzugsstellung der Mikrokontaktstruktur zum Zwecke der Optimierung von Kontakt bzw. Wirkungszusammenhang mit dem Nervengewebe durch eine extern festgelegte Einwirkung in einem zeitlich kontrollierten Ablauf bezüglich Bewegung und Kraft einstellbar ist.

11. Mikrokontaktstruktur nach einem der vorhergehenden Ansprüche **dadurch gekennzeichnet, dass** der Wechsel oder die Veränderung einer Vorzugsstellung der Mikrokontaktstruktur über eine chirurgische Vorrichtung oder über die Übermittlung von elektromagnetischen Signalen, Licht oder Ultraschall an die Mikrokontaktstruktur einstellbar ist.

12. Mikrokontaktstruktur nach einem der vorhergehenden Ansprüche, **dadurch gekennzeichnet, dass** der Wechsel der für die mechanische Verankerung der Mikrokontaktstruktur am Nervengewebe gewählten Vorzugsstellung zum Zwecke der Re-Explantation durch eine extern festgelegte Einwirkung in einem zeitlich kontrollierten Ablauf bezüglich Bewegung und Kraft dosiert einstellbar ist.

13. Mikrokontaktstruktur nach einem der vorhergehenden Ansprüche **dadurch gekennzeichnet, dass** die Mikrokontaktstruktur aus einem mehrschichtigen Substrat aufgebaut ist, wobei eine dem zu stimulierenden Nervengewebe zugewandte Schicht aus einem ersten Polymer hergestellt ist und penetrierende Elektroden aus Metall beinhaltet, das die anschließende Schicht bildet, eine weitere Schicht aus dem ersten Polymer und eine Schicht aus einem zweiten Polymer hergestellt ist.

14. Mikrokontaktstruktur nach Anspruch 13 **dadurch gekennzeichnet, dass** das zweite Polymer die Eigenschaft einer thermischen Ausdehnung relativ zum ersten Polymer und der Absorption von infraroter Strahlung aufweist, so dass durch Bestrahlung mit infraroter Strahlung eine definierte Volumenexpansion der Mikrokontaktstruktur einstellbar ist.

15. Mikrokontaktstruktur nach einem der Ansprüche 13 oder 14 **dadurch gekennzeichnet, dass** aufgrund der Materialeigenschaften der Mikrokontaktstruktur durch elektromagnetische Bestrahlung im ultravioletten Wellenlängenbereich strukturelle Übergänge im Material der Mikrokontaktstruktur einstellbar sind, die eine Volumenkontraktion der Mikrokontaktstruktur bewirken.

16. Mikrokontaktstruktur nach einem der Ansprüche 13 bis 15 **dadurch gekennzeichnet, dass** die Mikrokontaktstruktur aufgrund seiner Materialeigenschaften durch fokussierte Bestrahlung mit Infrarot-Licht an definierten Stellen verformbar und an das zu kontaktierende Nervengewebe anpassbar ist.

## Claims

1. Implantable microcontact structure for neuroprostheses for contacting nerve tissue, having a number of contact elements formed on at least one two-dimensional carrier which comprises at least two regions which are movable relative to each other and can assume at least two desired positions, namely a basic position and an operation position, wherein said microcontact structure can be set in a basic compact position for transportation from or to an implantation site, wherein the spatial extent of the microcontact structure is reduced by folding, nesting or rolling the regions which are movable relative to each another, and the microstructure can be set, for mechanical anchoring and for contacting of the nerve tissue at the implantation site, in an operation position with a planar or three-dimensional structure, wherein the spatial extent of the microcontact structure is enlarged by opening the folding, nesting or rolling of the regions movable relative to each other, **characterised in that** the microcontact structure is a microcontact structure for retinal implantation for a retina implant.

2. Microcontact structure according to claim 1, **characterised in that** said microcontact structure comprises a substrate of multi-layer construction which comprises memory properties in relation to the spatial arrangement of the microcontact structure.

3. Microcontact structure according to claim 2, **characterised in that** a change or alteration of the desired positions of the microcontact structure, on account of the memory properties of the microcontact structure and by external action before the implantation, can be actively controlled by external intervention or by external signals.

4. Microcontact structure according to one of the preceding claims, **characterised in that** the basic compact position of the microcontact structure is secured for surgical transportation by a transport lock which can be released by external intervention.

5. Microcontact structure according to claim 4, **characterised in that** the transport lock of the microcontact structure is constituted by a clamp, envelope or pinning.

6. Microcontact structure according to one of the claims 4 or 5, **characterised in that**, after removal of the transport lock, the microcontact structure unfolds independently on account of its memory properties and assumes a three-dimensional structure previously impressed in the material of the microcontact structure.

7. Microcontact structure according to one of the preceding claims, **characterised in that** the microcontact structure comprises raised microcontacts which engage with the tissue to be contacted in the operation position.

8. Microcontact structure according to one of the preceding claims, **characterised in that** the opening of the folding, nesting or rolling of the regions of the microcontact structure movable relative to each other takes place independently after release of the transport lock from the basic compact position into a desired position appropriate for the preparation of the mechanical anchoring through the release of spring forces, molecular conformation changes, pneumatic forces, hydraulic forces and / or electromagnetic forces at the transitions between the movable regions of the microcontact structure.

9. Microcontact structure according to one of the preceding claims, **characterised in that** the change or alteration of a desired position of the microcontact structure for the purpose of its mechanical anchoring to the nerve tissue can take place through an externally fixed intervention in a time-controlled sequence in relation to movement and force.

10. Microcontact structure according to one of the preceding claims, **characterised in that** the change or alteration of a desired position of the microcontact structure for the purpose of optimising contact or correlation of effect with the nerve tissue can take place through an externally fixed intervention in a time-controlled sequence in relation to movement and force.

11. Microcontact structure according to one of the preceding claims, **characterised in that** the change or alteration of a desired position of the microcontact structure can take place through a surgical device or through the transmission of electromagnetic signals, light or ultrasound to the microcontact structure.

12. Microcontact structure according to one of the preceding claims, **characterised in that** the change of the desired position selected for the mechanical anchoring of the microstructure to the nerve tissue can take place for the purpose of re-explantation through an externally fixed intervention in a time-controlled sequence in relation to movement and force.

13. Microcontact structure according to one of the preceding claims, **characterised in that** the microcontact structure comprises a substrate of multi-layer construction, wherein a layer facing the nerve tissue to be stimulated is composed of a first polymer and contains penetrating electrodes of metal which forms the adjoining layer, a further layer is produced from the first polymer and a layer from a second polymer.

14. Microcontact structure according to claim 13, **characterised in that** the second polymer has the property of a thermal expansion relative to the first polymer and the adsorption of infrared radiation, so that a defined volume expansion of the microcontact structure can take place through irradiation.with infrared radiation.

15. Microcontact structure according to one of the claims 13 or 14, **characterised in that**, on account of the material properties of the microcontact structure, structural transitions can take place in the material of the microcontact structure by means of electromagnetic radiation in the ultraviolet wavelength range, said structural transitions bringing about a volume contraction of the microcontact structure.

16. Microcontact structure according to one of the claims 13 to 15, **characterised in that** the microcontact structure, on account of its material properties, can be deformed by focussed irradiation with infrared light at defined points and matched to the nerve tissue to be contacted.

## Revendications

1. Structure de microcontact implantable pour des neuroprothèses destinées à entrer en contact avec des tissus nerveux, munie d'un certain nombre d'éléments de contact, qui sont formés au moins sur un support superficiel, qui présente au moins deux parties mobiles l'une par rapport à l'autre, qui peuvent adopter au moins deux positions privilégiées, à savoir une position de base et une position d'utilisation, ladite structure de microcontact pouvant être ajustée pour le transport depuis ou vers un site d'implantation dans une position de base compacte, dans laquelle l'étendue spatiale de la structure de microcontact peut être réduite par pliage, emboîtement ou enroulement des parties mobiles l'une par rapport à l'autre, et ladite structure de microcontact étant ajustable pour l'ancrage mécanique, ainsi que pour l'entrée en contact avec le tissu nerveux sur le site d'implantation dans une position d'utilisation dotée d'une structure plane ou tridimensionnelle, dans laquelle l'étendue spatiale de la structure de microcontact est agrandie par dépliage, désemboîtement ou déroulement des parties mobiles l'une par rapport à l'autre, **caractérisée en ce que** la structure de microcontact est une structure de microcontact pour l'implantation rétinienne d'un implant rétinien.

2. Structure de microcontact selon la revendication 1, **caractérisée en ce que** la structure de microcontact comprend un substrat constitué de plusieurs couches, qui présente des propriétés de mémoire de forme par rapport à l'agencement spatial de la structure de microcontact.

3. Structure de microcontact selon la revendication 2, **caractérisée en ce qu'**un changement ou une modification des positions privilégiées de la structure de microcontact peut être pilotée activement du fait des propriétés de mémoire de forme de la structure de microcontact et grâce à une action externe avant l'implantation, une intervention externe ou des signaux externes.

4. Structure de microcontact selon l'une quelconque des revendications précédentes, **caractérisée en ce que** la position de base compacte de la structure de microcontact est assurée pour le transport chirurgical grâce à une sécurité de transport, qui peut être désactivée par une intervention extérieure.

5. Structure de microcontact selon la revendication 4, **caractérisée en ce que** la sécurité de transport de la structure de microcontact est formée par une attache, une enveloppe ou une goupille.

6. Structure de microcontact selon la revendication 4 ou 5, **caractérisée en ce que** la structure de microcontact se déplie d'elle-même après désactivation de la sécurité de transport du fait de ses propriétés de mémoire de forme et adopte une structure tridimensionnelle imprimée au préalable au matériau de la structure de microcontact.

7. Structure de microcontact selon l'une quelconque des revendications précédentes, **caractérisée en ce que** la structure de microcontact présente des microcontacts en relief qui épousent le tissu devant entrer en contact avec elle en position d'utilisation.

8. Structure de microcontact selon l'une quelconque des revendications précédentes, **caractérisée en ce que** l'ouverture du pliage, de l'emboîtement ou de l'enroulement des parties mobiles l'une par rapport à l'autre de la structure de microcontact après désactivation de la sécurité de transport à partir de la position de base compacte aboutit d'elle-même à une position adaptée pour préparer l'ancrage mécanique grâce à la libération d'un mécanisme à ressort, de changements de conformation moléculaire, d'un mécanisme pneumatique, d'un mécanisme hydraulique et/ou d'un mécanisme électromagnétique lors des passages de transition des parties mobiles de la structure de microcontact.

9. Structure de microcontact selon l'une quelconque des revendications précédentes, **caractérisée en ce que** le changement ou la modification d'une position privilégiée de la structure de microcontact peut être ajusté à des fins d'ancrage mécanique sur le tissu nerveux grâce à une intervention définie de façon externe concernant le mouvement et la puissance dans le cadre d'un processus contrôlé dans le temps.

10. Structure de microcontact selon l'une quelconque des revendications précédentes, **caractérisée en ce que** le changement ou la modification de position privilégiée de la structure de microcontact est ajustable à des fins d'optimisation du contact ou du contexte opérationnel avec le tissu nerveux grâce à une intervention définie de façon externe concernant le mouvement et la puissance dans le cadre d'un processus contrôlé dans le temps.

11. Structure de microcontact selon l'une quelconque des revendications précédentes, **caractérisée en ce que** le changement ou la modification de position privilégiée de la structure de microcontact peut être ajusté grâce à un dispositif chirurgical ou l'émission de signaux électromagnétiques, de lumière ou d'ultrasons au niveau de la structure de microcontact.

12. Structure de microcontact selon l'une quelconque des revendications précédentes, **caractérisée en ce que** le changement de position privilégiée retenue pour l'ancrage mécanique de la structure de microcontact au tissu nerveux peut être ajusté de façon dosée à des fins d'explantation grâce à une intervention définie de façon externe concernant le mouvement et la puissance dans le cadre d'un processus contrôlé dans le temps.

13. Structure de microcontact selon l'une quelconque des revendications précédentes, **caractérisée en ce que** la structure de microcontact est constituée d'un substrat à plusieurs couches, dans lequel une couche orientée vers le tissu nerveux à stimuler est fabriquée à partir d'un premier polymère et contient des électrodes pénétrantes en métal, qui constitue la couche suivante, une autre couche est fabriquée à partir du premier polymère et une couche à partir d'un deuxième polymère.

14. Structure de microcontact selon la revendication 13, **caractérisée en ce que** le deuxième polymère présente une propriété d'expansion thermique par rapport au premier polymère et d'absorption de rayonnement infrarouge, de sorte que l'exposition à un rayonnement infrarouge permet une expansion de volume définie de la structure de microcontact.

15. Structure de microcontact selon la revendication 13 ou 14, **caractérisée en ce que**, du fait des propriétés des matériaux de la structure de microcontact, un rayonnement électromagnétique dans le domaine de longueur d'ondes ultraviolettes provoque des transitions structurelles du matériau de la structure de microcontact, qui entraînent une contraction du volume de la structure de microcontact.

16. Structure de microcontact selon l'une des revendications 13 à 15, **caractérisée en ce que** la structure de microcontact, du fait des propriétés de ses matériaux, est déformable en des endroits définis par exposition ciblée à une lumière infrarouge et peut être adaptée au tissu nerveux avec lequel elle doit entrer en contact.
